# EUROPEAN PATENT APPLICATION

(11) **EP 2 918 253 A1**
(43) Date of publication of application: **16.09.2015**
(21) Application number: 14164230.6
(22) Date of filing: 10.04.2014
(51) Int. Cl.: A61F 13/49, D03D 15/00

(54) **Reusable swim diaper**

(30) Priority: 13.03.2014 US 201461952564 P
(71) Applicant: i play. inc., Asheville, NC 28804 (US)
(72) Inventor: Cannon, Becky B., Asheville, NC North Carolina (US); Kubota, Emi, Asheville, NC North Carolina (US)
(74) Representative: Copsey, Timothy Graham

(57) **Abstract**

A reusable swim diaper, which includes a main torso part having an extent defined by an elastic-banded torso-receiving aperture at its upper end for receiving a torso, a pair of elastic-banded leg-receiving apertures at its lower end, and a crotch portion generally between said leg-receiving apertures. At least one joint extends between the torso-receiving aperture and one of the leg-receiving apertures, the joint being capable of selectively and repeatedly joining and separating adjacent portions of the main torso part. The main torso part also includes a bodyside inner layer of breathable wick away fabric having an extent corresponding to the extent of the main torso part, an absorbent terry cloth intermediate layer, and a waterproof outer layer having an extent corresponding to the extent of said main torso part. The layers are made of materials which are capable of withstanding laundering and are restored to substantially their original conditions by laundering.

## Description

### Technical Field

The invention relates to so called waterproof swim diapers, to be worn by infants and young children.

### Background Art

The reusable swim diaper disclosed and claimed herein is an improvement to the reusable swim diaper disclosed in Cannon et al patent number US 7,678,094, issued March 16, 2010 and titled "Reusable Swim Diaper."

### Disclosure of Invention

A reusable swim diaper is provided, which includes a main torso part having an extent defined by an elastic-banded torso-receiving aperture at its upper end for receiving a torso, a pair of elastic-banded leg-receiving apertures at its lower end, and a crotch portion generally between said leg-receiving apertures.

At least one joint extends between the torso-receiving aperture and one of the leg-receiving apertures, the joint being capable of selectively and repeatedly joining and separating adjacent portions of the main torso part. The main torso part also includes a bodyside inner layer of breathable wick away fabric having an extent corresponding to the extent of the main torso part, an absorbent terry cloth intermediate layer, and a waterproof outer layer having an extent corresponding to the extent of said main torso part. The layers are made of materials which are capable of withstanding laundering and are restored to substantially their original conditions by laundering.

### Brief Description Of The Drawings

FIG. 1 is a three-dimensional view of a swim diaper embodying the invention; and
FIG. 2 is a cross-sectional view taken on line 2-2 of FIG. 1.

### Best Mode for Carrying Out the Invention

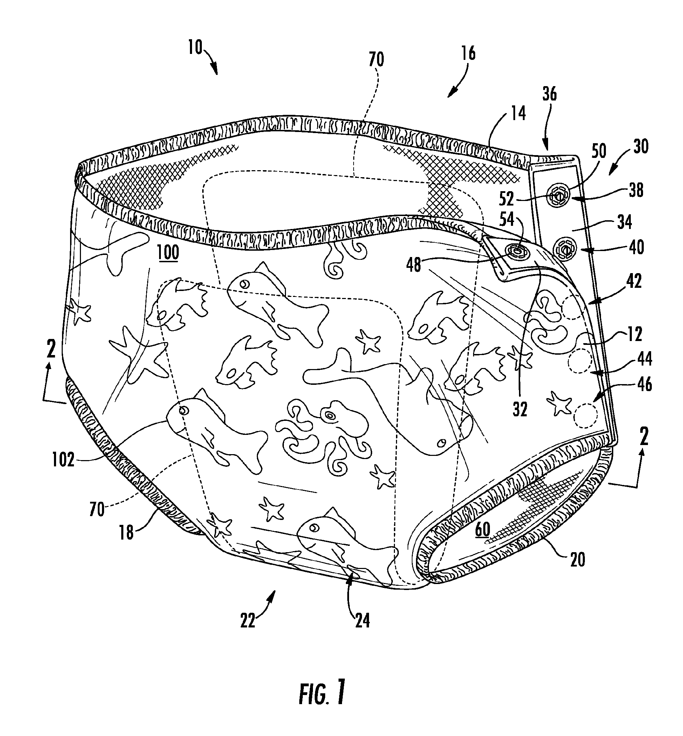

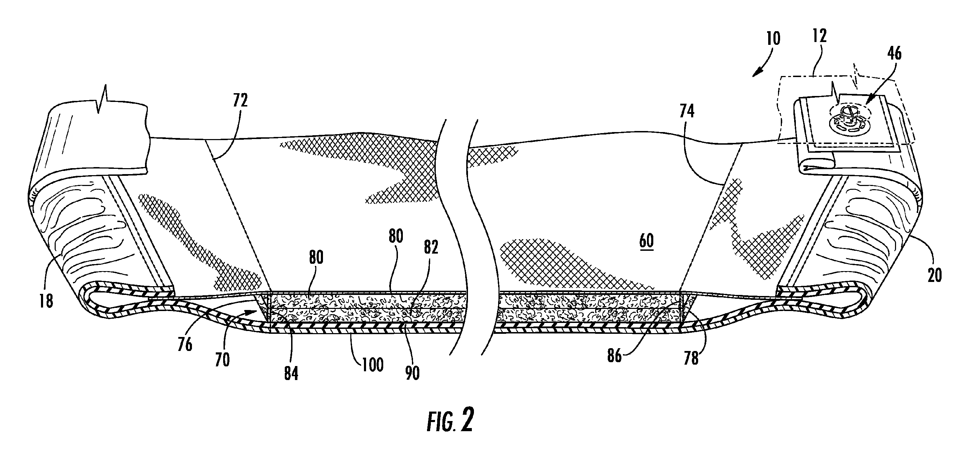

FIGS. 1 and 2 depict a swim diaper 10 of brief configuration embodying the invention. The swim diaper 10 includes a main torso part 12 having an extent defined by an elastic-banded torso-receiving aperture 14 at its upper end 16, and a pair of elastic-banded leg-receiving apertures 18 and 20 at its lower end 22. A crotch portion 24 is generally between the leg-receiving apertures 18 and 20.

To facilitate putting the swim diaper 10 on an infant or young child (not shown) and subsequently taking the swim diaper 10 off the infant or young child, at least one joint 30 extends between the torso-receiving aperture 14 and one of the leg-receiving apertures 18 or 20. The joint 30 is capable of selectively and repeatedly joining and separating front and rear adjacent portions 32 and 34 of the main torso part 12.

In the illustrated embodiment, the adjacent portions 32 and 34 of the main torso part 12 are overlapping, and the joint 30 includes a plurality 36 of snap fasteners 38, 40, 42, 44 and 46 along the overlapping portions 32 and 34. The number of snap fasteners of the plurality 36 can vary depending on the size of the swim diaper. For example, four or six snap fasteners may be employed.

In a conventional manner, representative snap fastener 38 includes a pair of interlocking discs 48 and 50 made of plastic and thermally bonded to respective overlapping portions. The disc 80 on the rear overlapping portion 34 has a circular lip 52 which snaps into a circular groove 54 on the front overlapping portion 32.

In the illustrated embodiment, the snap fasteners 38, 40, 42, 44 and 46 are carried on "snap tape" which is commercially available as a stock material. Thus each of the representative interlocking discs 48 and 50 is a rivet-like piece, in turn made of a hidden-side element and a visible-side element joined to each other through an aperture in the snap tape. The snap tape is attached to the front 32 and rear 34 portions of the main torso part 12 by conventional stitching.

The joint 30 is not absolutely liquid-tight (nor are seals provided by the torso receiving aperture 14 and the elastic banded leg receiving apertures 18 and 20), but the joint 30 is sufficiently tight so as to maintain the effectiveness of the swim diaper. It is particularly important that feces be contained.

The main torso part 12 is of multiple layer construction, and includes a bodyside inner layer 60 of breathable wick away fabric. A suitable breathable wick away fabric is a knitted polyester fabric. The bodyside inner layer 60 has an extent corresponding to the extent of the main torso part 12 itself, and accordingly is also defined by the torso-receiving aperture 14 and the leg-receiving apertures 18 and 20. The bodyside inner layer 60 is sewn at the edges of the apertures 14, 18 and 20.

Adjacent the bodyside inner layer 60 is an absorbent terrycloth intermediate layer 70. The absorbent terrycloth intermediate layer 70 has an extent corresponding to the crotch portion 24 of the main torso part 12. A suitable material for the absorbent terrycloth intermediate layer 70 is a polyester/polyamide fiber blend.

It is important that the terrycloth intermediate layer 70 have sufficient absorbency to absorb an anticipated volume of urine. In the illustrated embodiment, the absorbent terrycloth intermediate layer has a fabric weight of approximately 640 g/m², and is approximately 7 cm X 28 cm in size (if laid flat). The area of the absorbent terrycloth intermediate layer 70 is accordingly approximately 200 cm² (rounded up from 196 cm²). The absorbent terrycloth intermediate layer 70 is sewn to the bodyside inner layer 60 along two stitching lines 72 and 74 immediately adjacent lateral edges 76 and 78 of the intermediate layer 70.

Rather than a single layer having a fabric weight of approximately 640 g/m², and particularly in view of the availability of stock materials, the absorbent terrycloth intermediate layer 70 takes the form of two sublayers 80 and 82, each having a fabric weight of approximately 320 g/m². Stitches 84 and 86 extend through both sublayers 80 and 82, as well as through the bodyside inner layer 60.

The main torso part 12 additionally has a waterproof outer layer 90, which also has an extent corresponding to the extent of the main torso part 12.

The waterproof outer layer 90 is sewn at the edges of the torso-receiving aperture 14 and the leg-receiving apertures 18 and 20. A suitable material for the waterproof outer layer 90 is a coated woven nylon fabric. The waterproof outer layer 90, in combination with the elastic-banded apertures 14, 18 and 20, serves to minimize the flow of liquids, such as urine and swimming pool water, between the inside and the outside of the swim diaper 10, particularly between the absorbent terrycloth intermediate layer 70 and the outside environment.

For appearance purposes, the swim diaper 10 of FIGS. 1 and 2 additionally includes a decorative fabric visible layer 100, including representative decorative designs 102 printed thereon. The decorative fabric visible layer 100 can be of any suitable fabric, such as a nylon fabric. In the brief configuration embodiment 10 of FIGS. 1 and 2, the decorative fabric visible layer 100 has an extent corresponding to the extent of the main torso part 12, and is sewn to the main torso part 12 at the edge of the torso-receiving aperture 14, as well as at the edges of the leg-receiving apertures.

Significantly, all of the layers, the bodyside inner layer 60, the absorbent terrycloth intermediate layer 70, the waterproof outer layer 90 and the decorative fabric visible layer 100 are made of materials which are capable of withstanding laundering, and are restored to substantially their original condition by laundering. Thus, the swim diaper 10 is reusable, in contrast to disposable swim diapers. The swim diaper 10 is viewed as any other garment which is regularly laundered and worn again.

It will further be appreciated that the joint 30 extending between the torso-receiving aperture 14 and one of the leg-receiving apertures 18 or 20, with its snap fasteners 38, 40, 42, 44 and 46, facilitates putting the swim diaper 10 on an infant or young child and subsequently taking the swim diaper 10 off the infant or young child.

While particular embodiments of the invention have been illustrated and described herein, it is realized that numerous modifications and changes will occur to those skilled in the art. It is therefore to be understood that the appended claims are intended to cover all such modifications and changes as fall within the true spirit and scope of the invention.

### Industrial Applicability

The way in which the invention is capable of being exploited and the way in which it can be made and used will be apparent from the foregoing.

## Claims

1. A reusable swim diaper, comprising:
a main torso part having an extent defined by an elastic-banded torso-receiving aperture at its upper end, a pair of elastic-banded leg-receiving apertures at its lower end, and a crotch portion generally between said leg-receiving apertures, said main torso part including
at least one joint extending between said torso-receiving aperture and one of said leg-receiving apertures, said joint being capable of selectively and repeatedly joining and separating adjacent portions of said main torso part; and said main torso part including
a bodyside inner layer of breathable wick away fabric, said bodyside inner layer having an extent corresponding to the extent of said main torso part,
an absorbent terry cloth intermediate layer, and
a waterproof outer layer, said waterproof outer layer having an extent corresponding to the extent of said main torso part;
said layers being made of materials which are capable of withstanding laundering and are restored to substantially their original conditions by laundering.

2. The reusable swim diaper of claim 1, wherein:
said adjacent portions of said main torso part are overlapping; and
said joint comprises a plurality of snap fasteners along said overlapping portions.

3. The reusable swim diaper of claim 1 or Claim 2, wherein said absorbent terry cloth intermediate layer has an extent corresponding to said crotch portion.

4. The reusable swim diaper of any preceding claim, which further comprises a decorative fabric visible layer over said main torso part, said decorative fabric layer also made of a material which is capable of withstanding laundering and is restored to substantially its original condition by laundering.

5. The reusable swim diaper of claim 4, wherein said decorative fabric visible layer has an extent corresponding to the extent of said main torso part.

6. The reusable swim diaper of claim 4, wherein said decorative fabric visible layer has an extent greater than the extent of said main torso part so as to extend part way down the legs of a wearer.

7. The reusable swim diaper of any preceding claim, wherein said absorbent terry cloth intermediate layer has a fabric weight of approximately 640 g/m².

8. The reusable swim diaper of any preceding claim, wherein said absorbent terry cloth intermediate layer has an extent corresponding to said crotch portion, and an area of approximately 200 cm².

9. The reusable swim diaper of any preceding claim, wherein said absorbent terry cloth intermediate layer comprises two sublayers each having a fabric weight of in the order of 320 g/m².

10. The reusable swim diaper of any preceding claim, wherein said absorbent terry cloth intermediate layer comprises a polyester/polyamide fiber blend.

11. The reusable swim diaper of any preceding claim, wherein said bodyside inner layer comprises a knitted polyester fabric.

12. The reusable swim diaper of any preceding claim, wherein said waterproof outer layer comprises a coated woven nylon fabric.

13. The reusable swim diaper of any preceding claim, wherein said waterproof outer layer comprises a coated woven nylon fabric.
